# EUROPÄISCHE PATENTSCHRIFT

(11) **EP 0 367 164 B1**
(45) Veröffentlichungstag und Bekanntmachung des Hinweises auf die Patenterteilung: **24.02.1993**
(21) Anmeldenummer: 89120057.8
(22) Anmeldetag: 28.10.1989
(51) Int. Cl.: C07C 211/63, C07F 5/02, C07D 213/04

(54) **Ammonium- und Immoniumverbindungen und Verfahren zu ihrer Herstellung**
Ammonium and immonium compounds and process for their preparation
Composés d'ammonium et d'immonium et procédé de leur préparation

(30) Priorität: 03.11.1988 DE 3837344; 17.01.1989 DE 3901153
(43) Veröffentlichungstag der Anmeldung: 09.05.1990
(73) Patentinhaber: HOECHST AKTIENGESELLSCHAFT, 65926 Frankfurt am Main (DE)
(72) Erfinder: Gitzel,Jörg, Dr., D-6234 Hattersheim am Main (DE); Macholdt, Hans-Tobias, Dr., D-6100 Darmstadt (DE); Wehowsky, Frank, Dr., D-6272 Niedernhausen (DE); Prossel, Günther, Dr., D-8269 Burgkirchen (DE)

(56) Entgegenhaltungen:
- EP-A- 0 153 885
- DE-A- 2 244 297
- GB-A- 2 056 699
- US-A- 3 535 381

## Beschreibung

Gegenstand der vorliegenden Erfindung sind neue Ammonium-und Immoniumverbindungen und Verfahren zu ihrer Herstellung.

Die vorliegende Erfindung betrifft im einzelnen neue Ammoniumverbindungen der allgemeinen Formel (I) 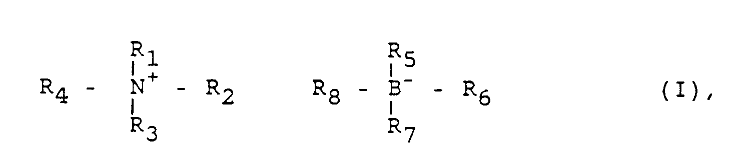
in welcher mindestens einer der Reste R₁ bis R₄ einen geradkettigen fluorhaltigen ungesättigten und/oder gesättigten Alkylrest mit 4 bis 30 C-Atomen und höchstens drei der Reste R₁ bis R₄ unabhängig voneinander Wasserstoffatome oder geradkettige oder verzweigte Alkyl-oder Hydroxyalkylreste mit 1 bis 30 C-Atomen, und R₅ bis R₈ Arylreste, wie beispielsweise Phenyl- oder Naphthylreste, ferner Alkylarylreste, wie beispielsweise den Toluylrest, Halogenarylreste, wie beispielsweise Fluorphenyl- oder Chlorphenylreste, oder Fluoratome bedeuten, sowie Gemische dieser Verbindungen, und Immoniumverbindungen der allgemeinen Formel (II) 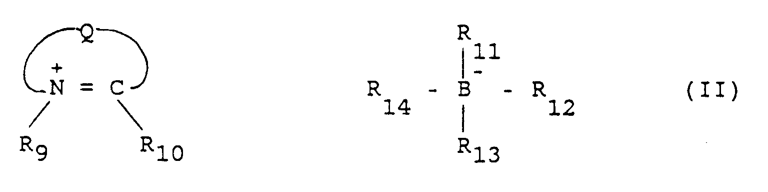
in welcher Q zusammen mit dem Bestandteil 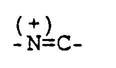
ein ein-oder mehrkerniges Ringsystem mit 4 bis 17 C-Atomen, das zusätzlich durch 1 bis 4 Heteroatome, wie beispielsweise Stickstoff-, Sauerstoff- oder Schwefelatome, unterbrochen sein und 2 bis 9 Doppelbindungen enthalten kann, bildet, welches in beliebiger Position durch Fluor-, Chlor-, Brom-oder Jodatome, Alkyl(C₁-C₆)-, Alkoxy(C₁-C₆)-, Nitro- oder Aminogruppen substituiert sein kann, R₉ einen fluorhaltigen Alkyl(C₁-C₃₀)-rest, R₁₀ ein Wasserstoff-, Fluor-, Chlor-, Brom- oder Jodatom, eine Alkyl(C₁-C₆)-, Alkoxy(C₁-C₆)-, Nitro- oder Aminogruppe und die Reste R₁₁ bis R₁₄ Arylreste, wie beispielsweise Phenyl- oder Naphthylreste, Alkylarylreste, wie beispielsweise den Toluylrest, Halogenarylreste, wie beispielsweise Fluorphenyl- oder Chlorphenylreste, oder Fluoratome bedeuten, sowie Gemische dieser Verbindungen.

Die Erfindung betrifft insbesondere solche Verbindungen der oben genannten allgemeinen Formel (I), in welcher mindestens einer der Reste R₁ bis R₄ einen geradkettigen fluorhaltigen ungesättigten und/oder gesättigten Alkylrest mit 4 bis 14 C-Atomen und höchstens drei der Reste R₁ bis R₄ unabhängig voneinander geradkettige oder verzweigte Alkyl(C₁-C₄)- oder Hydroxyalkyl(C₁-C₄)-reste bedeuten, und R₅ bis R₈ Phenyl-, Naphthyl-, p-Chlorphenyl-, p-Toluylreste oder Fluoratome bedeuten, sowie Verbindungen der genannten allgemeinen Formel (II), in welcher Q zusammen mit dem Bestandteil 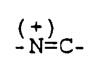
ein ein- oder mehrkerniges Ringsystem mit 4 bis 10 C-Atomen, das zusätzlich durch 1 bis 4 Heteroatome, wie beispielsweise Stickstoff-, Sauerstoff-oder Schwefelatome unterbrochen sein und 2 bis 5 Doppelbindungen enthalten kann, bildet, welches in beliebiger Position durch Fluor-, Chlor-, Brom- oder Jodatome, Alkyl(C₁-C₆)-, Alkoxy(C₁-C₆)-, Nitro- oder Aminogruppen substituiert sein kann, R₉ ein fluorhaltiger Alkyl(C₁-C₁₄)-rest, R₁₀ ein Wasserstoff-, Fluor-, Chlor-, Brom- oder Jodatome, eine Alkyl(C₁-C₆)-, Alkoxy(C₁-C₆)-, Nitro- oder Aminogruppe, und die Reste R₅ bis R₈ bzw. R₁₁ bis R₁₄ Phenyl-, p-Chlorphenyl-, p-Toluyl-, Naphthylreste oder Fluoratome bedeuten, sowie Gemische dieser Verbindungen.

Die Erfindung betrifft ganz besonders Verbindungen der oben genannten allgemeinen Formel (I), in welcher mindestens einer der Reste R₁ bis R₄ die Gruppe -CH₂-CH=CF-Rf (Rf = C₅F₁₁-C₁₁F₂₃), die Gruppe -CH₂-CH=CF-CₙF₂ₙ₊₁ mit n= 5, 7, 9 oder die Gruppe -CH₂-CH=CF-C₇F₁₅ und höchstens drei der Reste R₁ bis R₄ unabhängig voneinander eine Methyl-, Ethyl-, Butyl- oder Hydroxyethylgruppe bedeuten, sowie Verbindungen der oben genannten allgemeinen Formel (II), in welcher Q zusammen mit dem Bestandteil 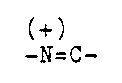
ein Pyridin-, Pyrazin- oder Chinolinringsystem bildet, R₉ eine C₈F₁₇-CH₂-CH₂-Gruppe, R₁₀ ein Wasserstoffatom und die Reste R₅ bis R₈ bzw. R₁₁ bis R₁₄ Phenyl-, p-Chlorphenyl-, p-Toluyl- oder Naphthylreste oder Fluoratome bedeuten, sowie Gemische dieser Verbindungen.

An Einzelverbindungen oder Gemischen von Verbindungen der allgemeinen Formeln (I) bzw. (II) sein beispielsweise folgende genannt:

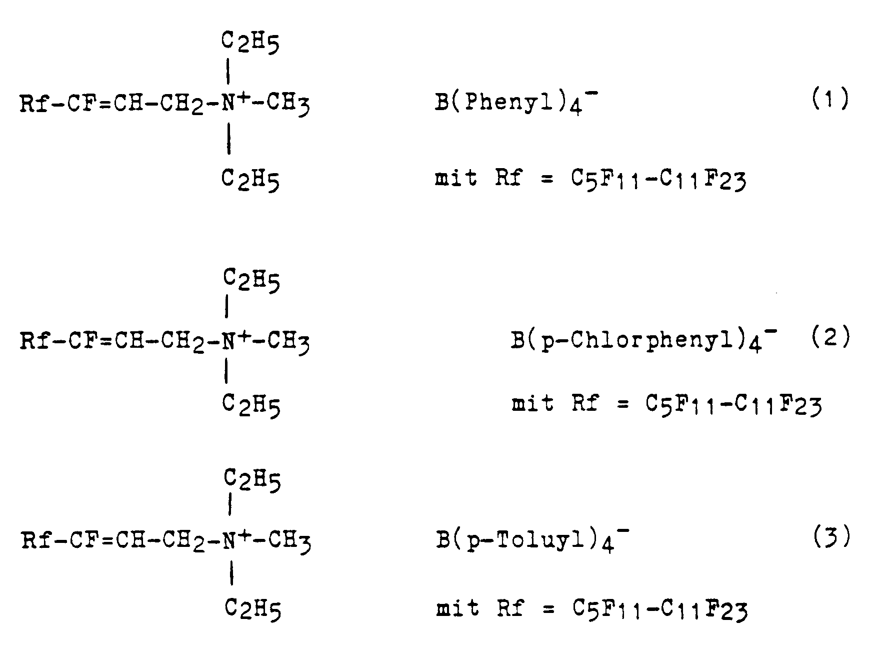

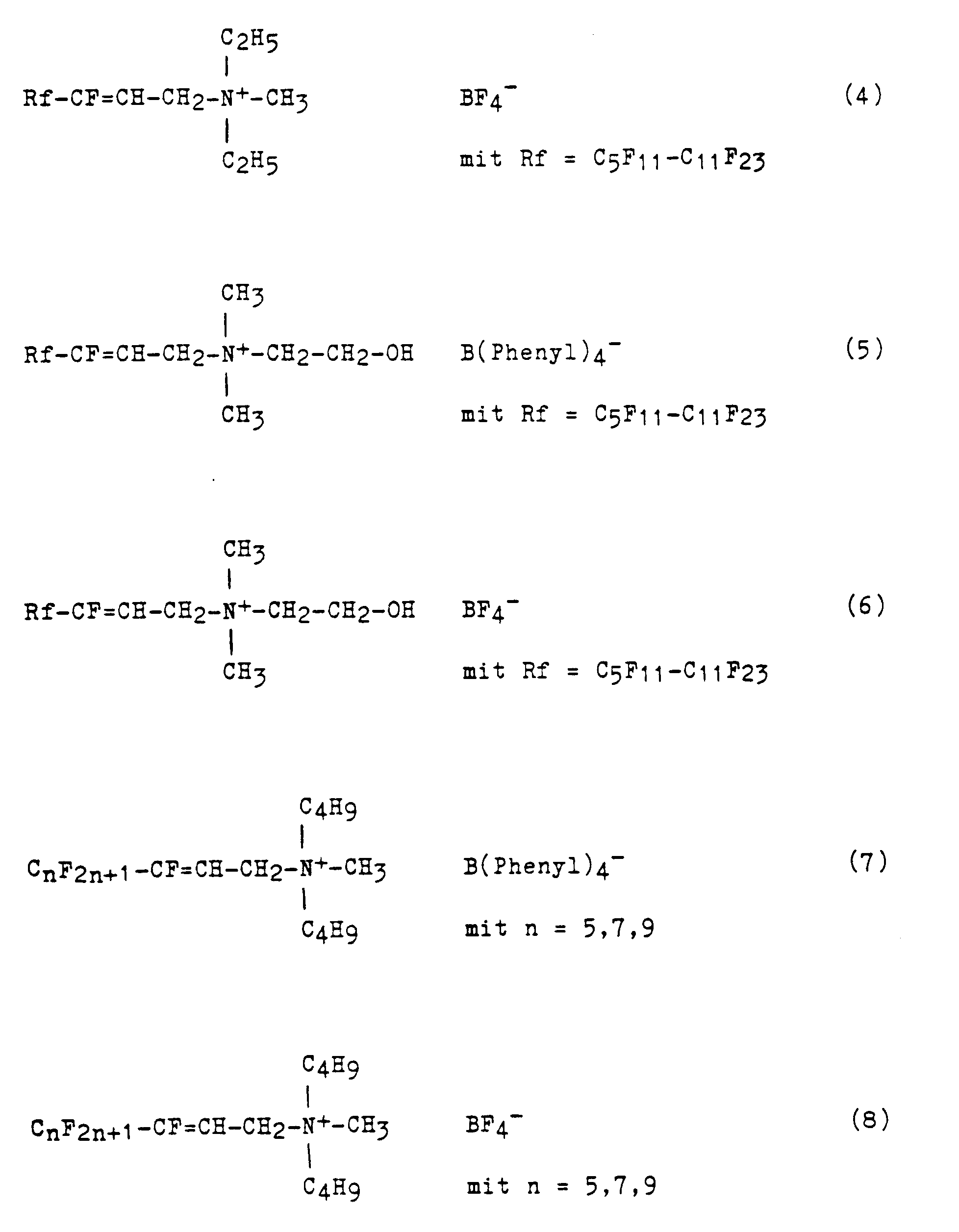

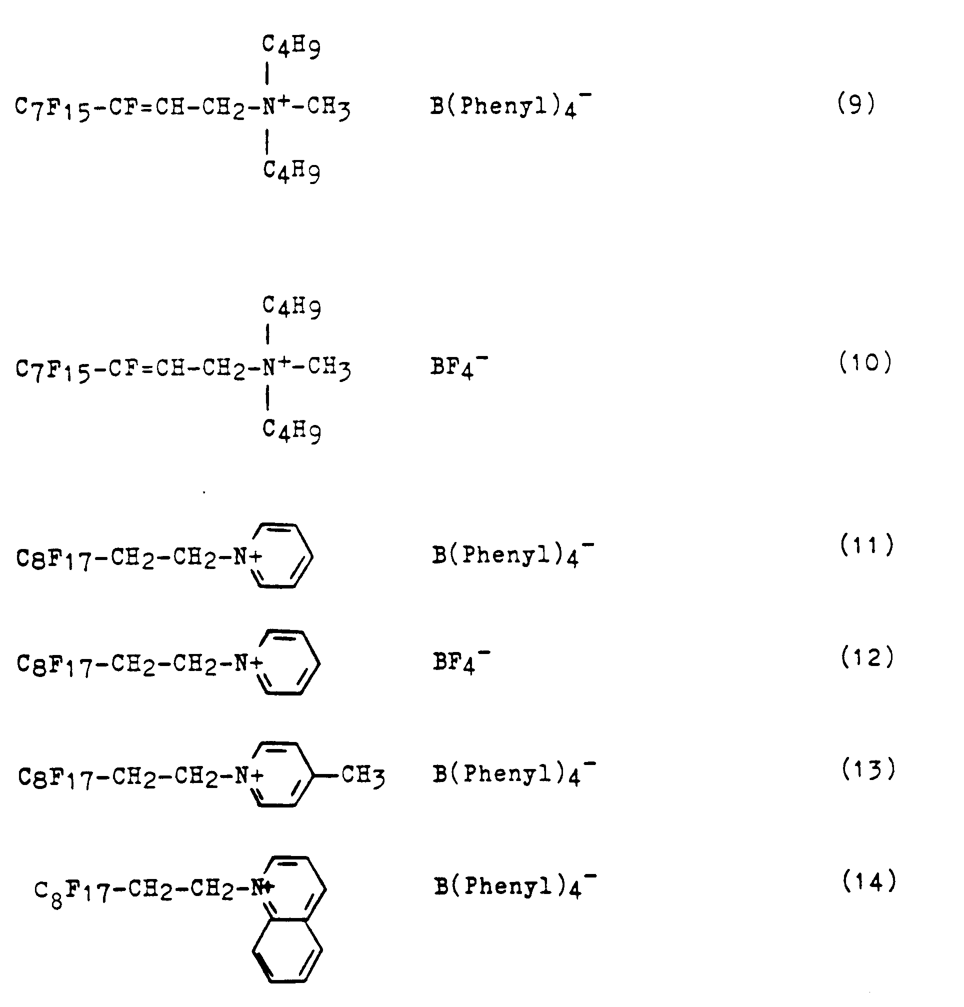

Die Ammonium- bzw. Immoniumverbindungen und Gemische aus Ammonium- bzw. Immoniumverbindungen der genannten allgemeinen Formeln (I) bzw. (II) können durch Umsetzen der Ammoniumverbindungen sowie Gemischen aus Ammoniumverbindungen der allgemeinen Formel (III) 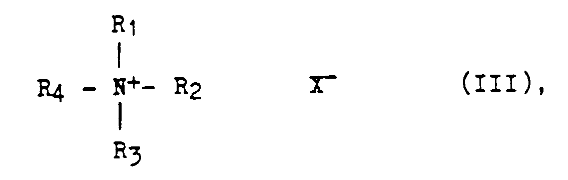
in welcher mindestens einer der Reste R₁ bis R₄ einen geradkettigen fluorhaltigen ungesättigten und/oder gesättigten Alkylrest mit 4 bis 30 C-Atomen und höchstens drei der Reste R₁ bis R₄ unabhängig voneinander Wasserstoffatome oder geradkettige oder verzweigte verzweigte Alkyl- oder Hydroxyalkylreste mit 1 bis 30 C-Atomen und X⁻ ein Halogenanion, beispielsweise ein Chlor-, Brom- oder Jodanion, oder ein Methylsulfatanion bedeuten, sowie durch Umsetzen von Immoniumverbindungen bzw. Gemischen aus Immoniumverbindungen der allgemeinen Formel (IV) 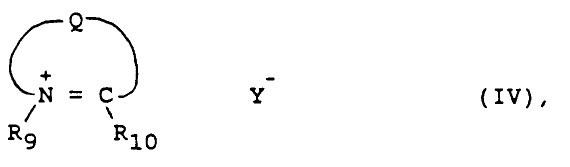
in welcher Q zusammen mit dem Bestandteil 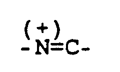
ein ein-oder mehrkerniges Ringsystem mit 4 bis 17 C-Atomen, das zusätzlich durch 1 bis 4 Heteroatome, vorzugsweise Stickstoff-, Sauerstoff- oder Schwefelatome unterbrochen sein und 2 bis 9 Doppelbindungen enthalten kann, bildet, welches in beliebiger Position durch Fluor-, Chlor-, Brom- oder Jodatome, eine Alkyl(C₁-C₆)-, Alkoxy(C₁-C₆)-, Nitro- oder Aminogruppe substituiert sein kann, R₉ einen fluorhaltigen Alkyl(C₁-C₃₀)-rest, R₁₀ ein Wasserstoff- Fluor-, Chlor-, Brom- oder Jodatom, eine Alkyl(C₁-C₆)-, Alkoxy(C₁-C₆)-, Nitro- oder Aminogruppe und Y⁻ ein Halogenanion, beispielsweise ein Chlor-, Brom- oder Jodanion, oder ein Methylsulfatanion bedeuten (die Synthese der Verbindungen der genannten allgemeinen Formeln III und IV wird in US-PS 3 535 381, DE-OS 1 922 277, DE-OS 2 244 297 und DE-OS 3 306 933 beschrieben), mit einem Boratsalz in Wasser oder Gemischen aus Wasser und organischem Lösungsmittel, wie beispielsweise Isopropanol, Isobutanol oder Methylisobutylketon, bei Temperaturen von etwa 20°C bis etwa 90°C, vorzugsweise von etwa 50°C bis etwa 80°C dargestellt werden.

Die Verbindungen und Verbindungsgemische der allgemeinen Formeln (I) bzw. (II) fallen hierbei in guter Ausbeute und Reinheit an und können direkt aus dem Reaktionsmedium durch Abfiltrieren isoliert werden.

So werden beispielsweise die vorstehenden Verbindungen der Formeln (1) bis (14) durch Umsetzen der nachstehenden Ausgangsverbindungen (15) bis (21) 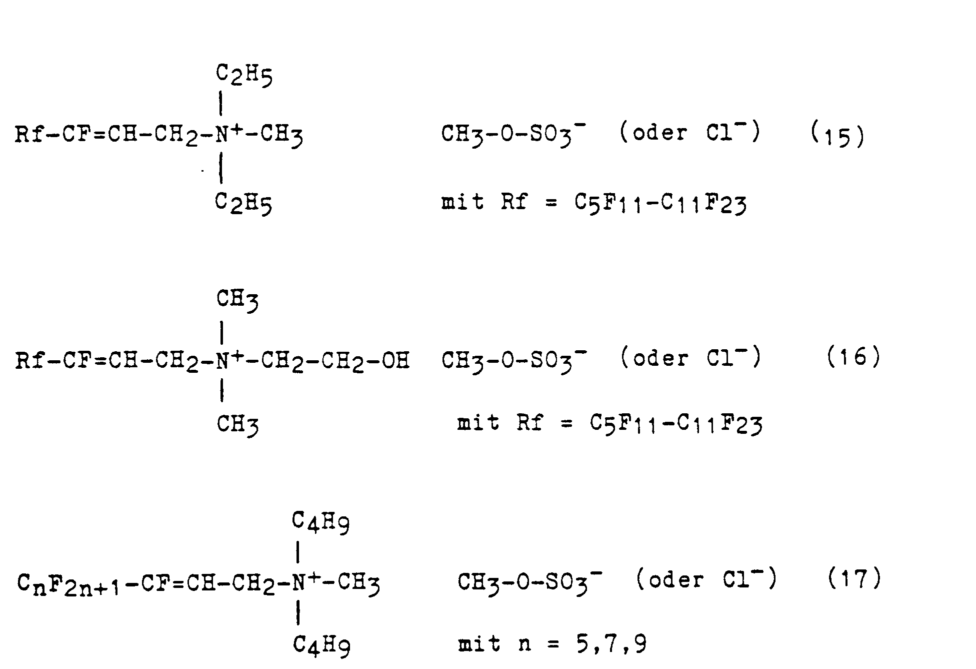 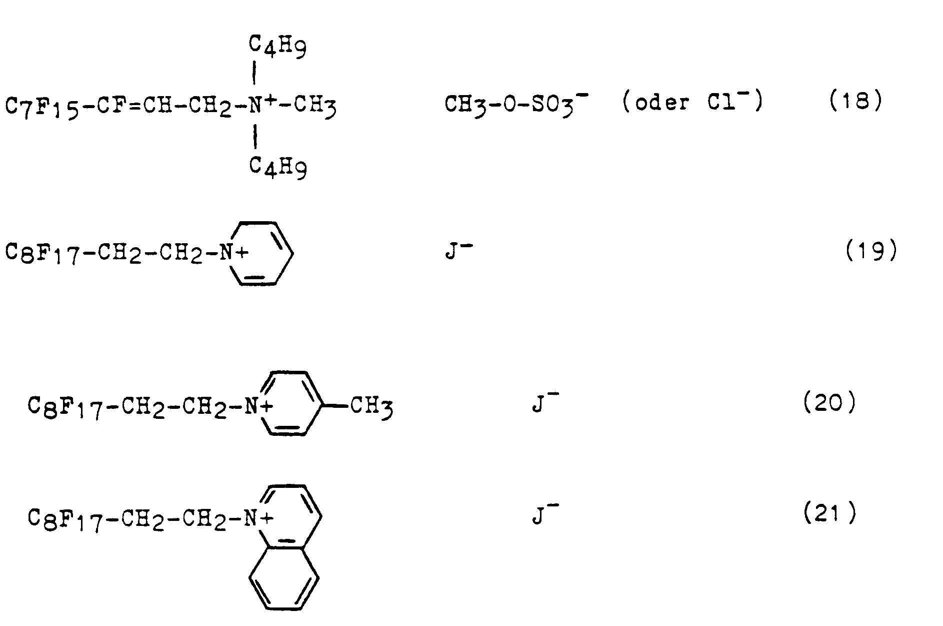
mit Natrium-tetra-phenylborat, Natrium-tetra-p-chlorphenylborat, Natrium-tetra-p-toluylborat oder Natrium-tetra-fluoroborat dargestellt (siehe nachstehende Herstellungsbeispiele 1 bis 6). Natrium-tetra-p-chlorphenylborat und Natrium-tetra-p-toluylborat wurden nach der Vorschrift von H.Holzapfel und C. Richter, J. Prakt. Chem. 26 (1964), 15-23, hergestellt. Die erfindungsgemäßen Ammonium- und Immoniumverbindungen sind hervorragend geeignet für den Einsatz als Ladungssteuermittel in elektrophotographischen Tonern und Entwicklern für elektrophotographische Aufzeichnungsverfahren.

Die nachstehenden Herstellungsbeispiele dienen zur Erläuterung der Erfindung, ohne diese darauf zu beschränken.

### Herstellungsbeispiel 1

Zu 40 ml einer 0,5 molaren wäßrigen Lösung (0,020 Mol) der Verbindung der weiter oben genannten Formel (15) (Molekulargewicht 672) werden 160 ml Wasser gegeben und dann über einen Zeitraum von 15 bis 20 Minuten 22 ml einer 1,0 M wäßrigen Lösung von Natrium-tetraphenylborat (0,022 Mol) unter kräftigem Rühren zugetropft. Es wird mit Wasser auf 500 ml aufgefüllt, auf 50°C erwärmt und der dicke weiße Niederschlag heiß abgesaugt. Das Produkt wird mit Wasser gründlich gewaschen und bei 50°C im Umluftschrank getrocknet.
- Ausbeute:: 16,9 g (96,0 % d. Th.) der Verbindung (1)
- Molekulargewicht:: 880
- Schmelzpunkt:: 154-156°C

| | | | | |
|---|---|---|---|---|
| Elementaranalyse: | ber. 4,1 % H, | 1,6 % N, | 1,2 % B | |
| | gef. 4,1% H, | 1,6 % N, | 1,1 % B, | 0,05% Wasser |

- 1H-NMR(in DMSO-d6):: 1,20 (Triplett, 6 Methyl-H), 2,97 (Singulett, 3 Methyl-H), 3,30 (Quartett, 4-Methylen-H), 4,25 (Dublett, 2 Allyl-H), 6,61 (Dublett von Tripletts, 1 Vinyl-H), 7,01 (Multiplett, 20 Phenyl-H) ppm.

### Herstellungsbeispiel 2

Es wurde, wie in Herstellungsbeispiel 1 beschrieben, gearbeitet, mit dem Unterschied, daß statt Natrium-tetra-phenylborat Natrium-tetra-p-chlor-phenylborat eingesetzt wurde.
- Ausbeute:: 15,4 g (75,6 % d. Th.) der Verbindung der weiter oben genannten Formel (2)
- Molekulargewicht:: 1018
- Schmelzpunkt:: 63-64°C

| | | | | | |
|---|---|---|---|---|---|
| Elementaranalyse: | ber. 3,1% H | 1,4% N | 1,1% B | 13,9% Cl | |
| | gef. 3,4% H | 1,5% N | 1,1% B | 14,5% Cl | 0,07 % H₂O |

- 1H-NMR(in DMSO-d6):: 1,23 (Triplett, 6 Methyl-H), 3,01 (Singulett, 3 Methyl-H), 3,36 (Quartett, 4 Methylen-H), 4,28 (Dublett, 2 Allyl-H), 6,63 (Dublett von Tripletts, 1 Vinyl-H), 7,04 (Multiplett, 16 p-Chlorphenyl-H) ppm.

### Herstellungsbeispiel 3

Es wurde, wie in Herstellungsbeispiel 1 beschrieben, gearbeitet, mit dem Unterschied, daß statt Natrium-tetra-phenylborat Natrium-tetra-p-toluylborat eingesetzt und das Lösungsmittel durch Isopropanol/Wasser (1:1 Volumenteile) ersetzt wurde.
- Ausbeute:: 11,5 g (61,2 % d. Th.) der Verbindung der weiter oben genannten Formel (3)
- Molekulargewicht:: 936
- Schmelzpunkt:: 151-152°C

| | | | | |
|---|---|---|---|---|
| Elementaranalyse: | ber. 4,7% H | 1,5% N | 1,2% B | |
| | gef. 5,0% H | 1,9% N | 1,2% B | 0,07% H₂O |

- 1H-NMR(in DMSO-D6):: 1,21 (Triplett, 6-Methyl-H), 2,15 (Singulett, 3 Toluylmethyl-H), 2,97 (Singulett, 3 Methyl-H), 3,33 (Quartett, 4 Methylen-H), 4,23 (Dublett, 2 Allyl-H), 6,60 (teilweise überlagertes Dublett von Tripletts, 1 Vinyl-H), 6,88 (Multiplett, 16 p-Toluyl-H) ppm.

### Herstellungsbeispiel 4

Es wurde, wie in Herstellungsbeispiel 1 beschrieben, gearbeitet, mit dem Unterschied, daß statt Natrium-tetra-phenylborat Natrium-tetra-fluoroborat eingesetzt wurde.
- Ausbeute:: 12,3 g (95,0 % d. Th.) der Verbindung der weiter oben genannten Formel (4)
- Molekulargewicht:: 648
- Schmelzpunkt:: 202°C

| | | | | |
|---|---|---|---|---|
| Elementaranalyse: | ber. 2,5 % H, | 2,2 % N, | 1,7 % B | |
| | gef. 2,7 % H, | 2,3 % N, | 1,8 % B, | 0,5 % Wasser |

- 1H-NMR (in DMSO-d6):: 1,24 (Triplett, 6 Methyl-H),3,02 (Singulett, 3 Methyl-H), 3,37 (Quartett, 4 Ethyl-H), 4,28 (Dublett, 2 Allyl-H), 6,63 (Dublett von Tripletts, 1 Vinyl-H) ppm.

### Herstellungsbeispiel 5

7,8 g (0,021 Mol) der Verbindung (19) werden in 200 ml warmem Isobutanol gelöst. Dann werden 100 ml Wasser zugesetzt und anschließend 4,1 g (0,012 Mol) Natrium-tetra-phenylborat unter Rühren langsam zugegeben. Es wird 1 Stunde kräftig gerührt und dann das Produkt abgenutscht, mit Isobutanol/Wasser (1:1 Volumenteile), dann mit Wasser gewaschen und bei 50 °C im Vakuum getrocknet.
- Ausbeute:: 9,1 g (89,7 % d. Th.) der Verbindung der weiter oben genannten Formel (11)
- Molekulargewicht:: 845
- Schmelzpunkt:: 163°C

| | | | | | | |
|---|---|---|---|---|---|---|
| Elementaranalyse: | ber. 55,4 % C, | 3,5 % H, | 1,7 % N, | 1,3 % B, | 38,2 % F | |
| | gef. 55,2 % C, | 3,3 % H, | 1,6 % N, | 1,2 % B, | 36,8 % F, | 0,19 % Wasser |

- 1H-NMR (in DMSO-d6):: 3,25 (Multiplett, 2 Methylen-H), 4,97 (Triplett, 2 Methylen-H), 7,00 (Multiplett, 20 Phenyl-H), 8,59 (Multiplett, 5 Pyridyl-H) ppm.

### Herstellungsbeispiel 6

11,1 g (0,017 Mol) der Verbindung der weiter oben genannten Formel (19) werden in 400 ml heißem Wasser gelöst und dann 1,9 g (0,017 Mol) Natriumtetrafluoroborat unter Rühren langsam zugegeben. Es wird 1 Stunde kräftig gerührt, das Produkt heiß abgenutscht, mit heißem Wasser gewaschen und bei 50°C im Vakuum getrocknet.
- Ausbeute:: 8,7 g (83,5 % d.Th.) der Verbindung der weiter oben genannten Formel (12)
- Molekulargewicht:: 613
- Schmelzpunkt:: 169°C

| | | | | | |
|---|---|---|---|---|---|
| Elementaranalyse: | ber. 29,4 % C, | 1,5 % H, | 2,3 % N, | 1,8 % B | |
| | gef. 29,5 % C, | 1,6 % H, | 2,4 % N, | 1,5 % B | 0,06 % H₂O |

- 1H-NMR (in DMSO-d6):: 3,21 (Multiplett, 2 Methylen-H), 5,03 (Triplett, 2 Methylen-H), 8,64 (Multiplett, 5 Pyridyl-H) ppm.

Analog wurden die Einzelverbindungen bzw. Verbindungsgemische der weiter oben genannten Formeln (5) bis (10) sowie (13) und (14) erhalten.

## Patentansprüche

1. Ammoniumverbindungen der allgemeinen Formel (I) 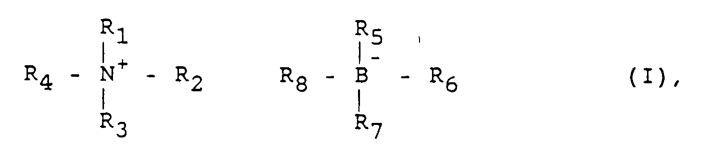 in welcher mindestens einer der Reste R₁ bis R₄ einen geradkettigen fluorhaltigen ungesättigten und/oder gesättigten Alkylrest mit 4 bis 30 C-Atomen und höchstens drei der Reste R₁ bis R₄ unabhängig voneinander Wasserstoffatome oder geradkettige oder verzweigte Alkyl-oder Hydroxyalkylreste mit 1 bis 30 C-Atomen, und R₅ bis R₈ Aryl-, Alkylaryl-, Halogenarylreste oder Fluoratome bedeuten, sowie Immoniumverbindungen der allgemeinen Formel (II), 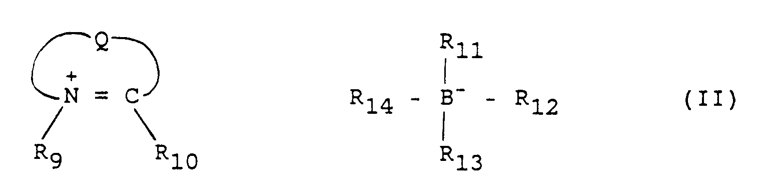 in welcher Q zusammen mit dem Bestandteil 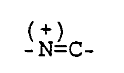 ein ein-oder mehrkerniges Ringsystem mit 4 bis 17 C-Atomen, das zusätzlich durch 1 bis 4 Heteroatome unterbrochen sein und 2 bis 9 Doppelbindungen enthalten kann, bildet, welches in beliebiger Position durch Fluor-, Chlor-, Brom- oder Jodatome, Alkyl(C₁-C₆)-, Alkoxy(C₁-C₆)-, Nitro- oder Aminogruppen substituiert sein kann, R₉ einen fluorhaltigen Alkyl(C₁-C₃₀)-rest, R₁₀ ein Wasserstoff-, Fluor-, Chlor-, Brom- oder Jodatom, eine Alkyl(C₁-C₆)-, Alkoxy(C₁-C₆)-, Nitro- oder Aminogruppe und die Reste R₁₁ bis R₁₄ Arylreste, Alkylarylreste, Halogenarylreste oder Fluoratome bedeuten, sowie Gemische dieser Verbindungen.

2. Verbindungen der in Anspruch 1 genannten allgemeinen Formel (I), in welcher mindestens einer der Reste R₁ bis R₄ einen geradkettigen fluorhaltigen ungesättigten und/oder gesättigten Alkylrest mit 4 bis 14 C-Atomen und höchstens drei der Reste R₁ bis R₄ unabhängig voneinander geradkettige oder verzweigte Alkyl(C₁-C₄)- oder Hydroxyalkyl(C₁-C₄)-reste und R₅ bis R₈ Phenyl-, Naphthyl-, p-Chlorphenyl-, p-Toluylreste oder Fluoratome bedeuten, sowie Verbindungen der in Anspruch 1 genannten allgemeinen Formel (II), in welcher Q zusammen mit dem Bestandteil 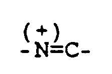 ein ein- oder mehrkerniges Ringsystem mit 4 bis 10 C-Atomen, das zusätzlich durch 1 bis 4 Heteroatome unterbrochen sein und 2 bis 5 Doppelbindungen enthalten kann, bildet, welches in beliebiger Position durch Fluor-, Chlor-, Brom- oder Jodatome, Alkyl(C₁-C₆)-, Alkoxy(C₁-C₆)-, Nitrooder Aminogruppen substituiert sein kann, R₉ ein fluorhaltiger Alkyl(C₄-C₁₄)-rest, R₁₀ ein Wasserstoff-, Fluor-, Chlor-, Brom- oder Jodatom, eine Alkyl(C₁-C₆)-, Alkoxy(C₁-C₆)-, Nitro- oder Aminogruppe, und die Reste R₅ bis R₈ bzw. R₁₁ bis R₁₄ Phenyl-, p-Chlorphenyl-, p-Toluyl-, Naphthylreste oder Fluoratome bedeuten, sowie Gemische dieser Verbindungen.

3. Verbindungen der in Anspruch 1 genannten allgemeinen Formel (I), in welcher mindestens einer der Reste R₁ bis R₄ die Gruppe -CH₂-CH=CF-Rf (Rf = C₅F₁₁-C₁₁F₂₃), die Gruppe -CH₂-CH=CF-CₙF₂ₙ₊₁ mit n = 5,7,9 oder die Gruppe -CH₂-CH=CF-C₇F₁₅ und höchstens drei der Reste R₁ bis R₄ unabhängig voneinander eine Methyl-, Ethyl-, Butyl- oder Hydroxyethylgruppe bedeuten, sowie Verbindungen der in Anspruch 1 genannten allgemeinen Formel (II), in welcher Q zusammen mit dem Bestandteil 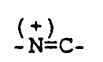 ein Pyridin-, Pyrazin-oder Chinolinringsystem bildet, R₉ eine C₈F₁₇-CH₂-CH₂-Gruppe, R₁₀ ein Wasserstoffatom und die Reste R₅ bis R₈ bzw. R₁₁ bis R₁₄ Phenyl-, p-Chlorphenyl-, p-Toluyl- oder Naphthylreste oder Fluoratome bedeuten, sowie Gemische dieser Verbindungen.

4. Verbindungen bzw. Gemische aus Verbindungen der Formel

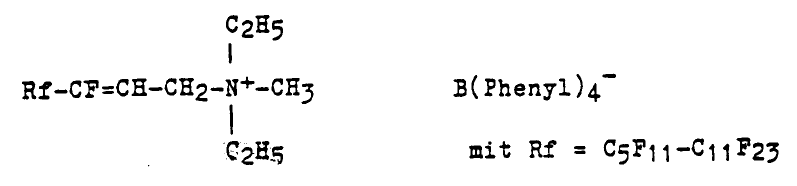

5. Verbindungen bzw. Gemische aus Verbindungen der Formel

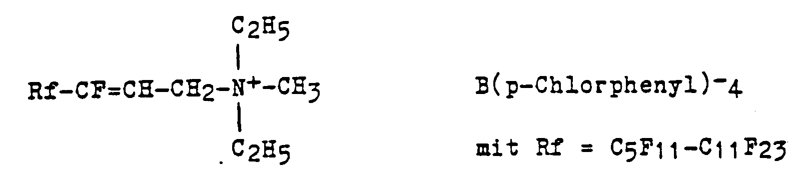

6. Verbindungen bzw. Gemische aus Verbindungen der Formel

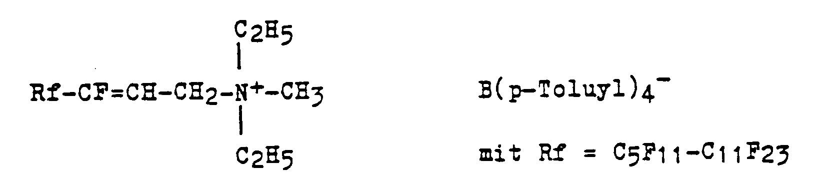

7. Verbindungen bzw. Gemische aus Verbindungen der Formel

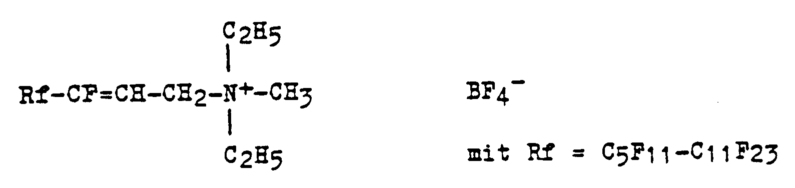

8. Verbindungen bzw. Gemische aus Verbindungen der Formel

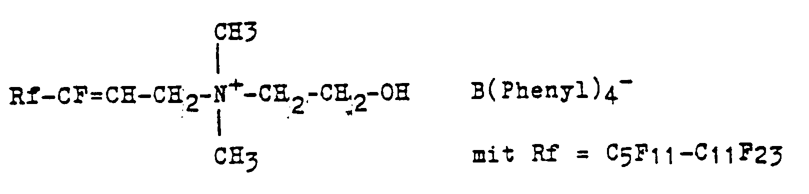

9. Verbindungen bzw. Gemische aus Verbindungen der Formel

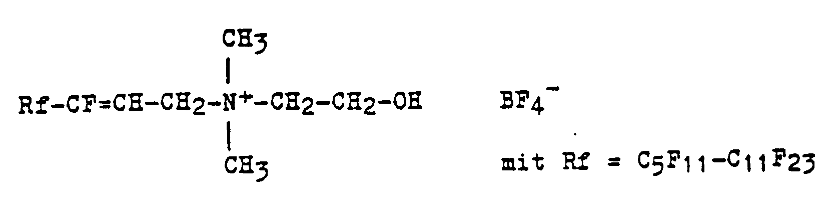

10. Verbindungen bzw. Gemische aus Verbindungen der Formel

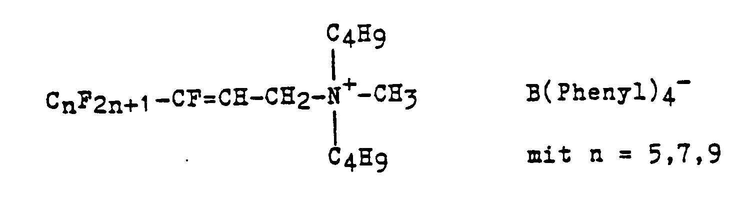

11. Verbindungen bzw. Gemische aus Verbindungen der Formel

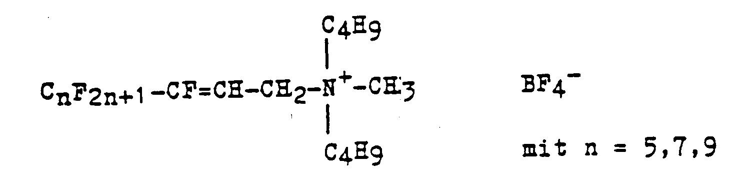

12. Verbindung der Formel

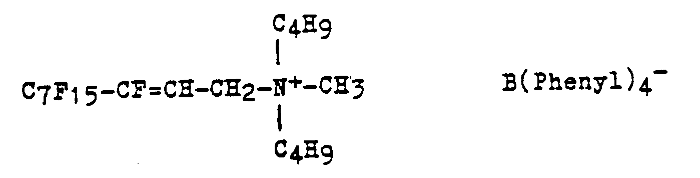

13. Verbindung der Formel

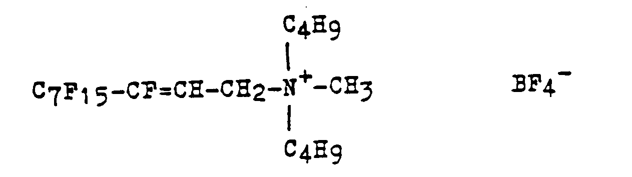

14. Verbindung der Formel

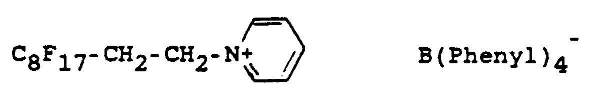

15. Verbindung der Formel

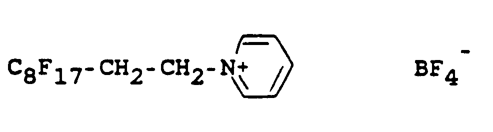

16. Verfahren zur Herstellung von Ammoniumverbindungen bzw. Gemischen aus Ammoniumverbindungen der in Anspruch 1 genannten allgemeinen Formel (I) sowie von Immoniumverbindungen bzw. Gemischen aus Immoniumverbindungen der in Anspruch 1 genannten allgemeinen Formel (II), dadurch gekennzeichnet, daß man Ammoniumverbindungen bzw. Gemische aus Ammoniumverbindungen der allgemeinen Formel (III) 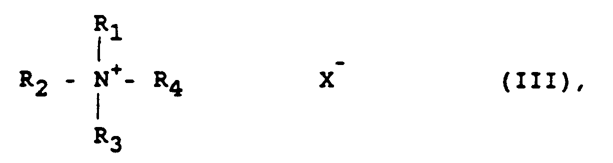 in welcher mindestens einer der Reste R₁ bis R₄ einen geradkettigen fluorhaltigen ungesättigten und/oder gesättigten Alkylrest mit 4 bis 30 C-Atomen und höchstens drei der Reste R₁ bis R₄ unabhängig voneinander Wasserstoffatome oder geradkettige oder verzweigte Alkyl- oder Hydroxyalkylreste mit 1 bis 30 C-Atomen und X⁻ ein Halogenanion oder ein Methylsulfatanion bedeuten, bzw. daß man Immoniumverbindungen bzw. Gemische aus Immoniumverbindungen der in Anspruch 1 genannten allgemeinen Formel (IV) 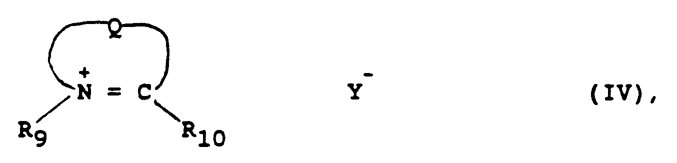 in welcher Q zusammen mit dem Bestandteil 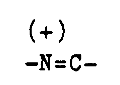 ein ein- oder mehrkerniges Ringsystem mit 4 bis 17 C-Atomen, das zusätzlich durch 1 bis 4 Heteroatome unterbrochen sein und 2 bis 9 Doppelbindungen enthalten kann, bildet, welches in beliebiger Position durch Fluor-, Chlor-, Brom- oder Jodatome, Alkyl(C₁-C₆)-, Alkoxy(C₁-C₆)-, Nitro- oder Aminogruppen substituiert sein kann, R₉ einen fluorhaltigen Alkyl(C₁-C₃₀)-rest, R₁₀ ein Wasserstoff-, Fluor-, Chlor-, Brom- oder Jodatome, eine Alkyl(C₁-C₆)-, Alkoxy-(C₁-C₆)-, Nitro- oder Aminogruppe und Y⁻ ein Halogenanion oder ein Methylsulfatanion bedeuten, mit einem Boratsalz in Wasser oder Gemischen aus Wasser und organischem Lösungsmittel bei Temperaturen von etwa 20°C bis etwa 90°C umsetzt.

17. Verfahren nach Anspruch 16, dadurch gekennzeichnet, daß in der dort genannten allgemeinen Formel (III) X⁻ und in der dort genannten allgemeinen Formel (IV) Y⁻ ein Chlor-, Brom- oder Jodanion bedeuten.

18. Verfahren nach mindestens einem der Ansprüche 16 und 17 dadurch gekennzeichnet, daß man in einem Gemisch aus Wasser und Isopropanol, Isobutanol oder Methylisobutylketon umsetzt.

19. Verfahren nach mindestens einem der Ansprüche 16 bis 18, dadurch gekennzeichnet, daß man mit Natrium-tetraphenylborat, Natrium-tetra-p-chlorphenylborat, Natrium-tetra-p-toluylborat oder Natrium-tetra-fluoroborat als Boratsalz umsetzt.

## Claims

1. An ammonium compound of the formula (I) 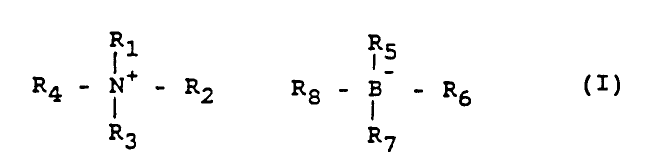 in which at least one of the radicals R₁ to R₄ denotes a straight-chain fluorine-containing unsaturated and/or saturated alkyl radical having 4 to 30 carbon atoms and not more than three of the radicals R₁ to R₄ independently of one another denote hydrogen atoms or straight-chain or branched alkyl or hydroxyalkyl radicals having 1 to 30 carbon atoms, and R₅ to R₈ denote aryl, alkylaryl or halogenoaryl radicals or fluorine atoms, or an iminium compound of the formula (II) 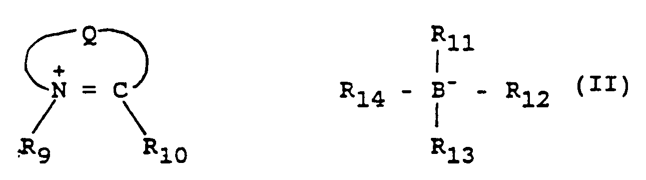 in which Q, together with the constituent 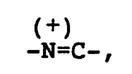 forms a mono- or polynuclear ring system having 4 to 17 carbon atoms, which can additionally be interrupted by 1 to 4 hetero atoms and contain 2 to 9 double bonds, and which can be substituted in any desired position by fluorine, chlorine, bromine or iodine atoms or alkyl(C₁-C₆), alkoxy(C₁-C₆), nitro or amino groups, R₉ denotes a fluorine-containing alkyl(C₁-C₃₀) radical, R₁₀ denotes a hydrogen, fluorine, chlorine, bromine or iodine atom or an alkyl(C₁-C₆), alkoxy(C₁-C₆), nitro or amino group and the radicals R₁₁ to R₁₄ denote aryl radicals, alkylaryl radicals, halogenoaryl radicals or fluorine atoms, or a mixture of these compounds.

2. A compound of the formula (I) as claimed in claim 1, in which at least one of the radicals R₁ to R₄ denotes a straight-chain fluorine-containing unsaturated and/or saturated alkyl radical having 4 to 14 carbon atoms and not more than three of the radicals R₁ to R₄ independently of one another denote straight-chain or branched alkyl(C₁-C₄) or hydroxyalkyl(C₁-C₄) radicals, and R₅ to R₈ denote phenyl, naphthyl, p-chlorophenyl, p-toluyl radicals or fluorine atoms, or a compound of the formula (II) mentioned in claim 1, in which Q, together with the constituent 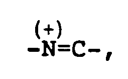 forms a mono- or polynuclear ring system having 4 to 10 carbon atoms, which can additionally be interrupted by 1 to 4 hetero atoms and contain 2 to 5 double bonds and which can be substituted in any desired position by fluorine, chlorine, bromine or iodine atoms or alkyl(C₁-C₆), alkoxy(C₁-C₆), nitro or amino groups, R₉ denotes a fluorine-containing alkyl(C₄-C₁₄) radical, R₁₀ denotes a hydrogen, fluorine, chlorine, bromine or iodine atom or an alkyl(C₁-C₆), alkoxy(C₁-C₆), nitro or amino group and the radicals R₅ to R₈ and R₁₁ to R₁₄ denote phenyl, p-chlorophenyl, p-toluyl or naphthyl radicals or fluorine atoms, or a mixture of these compounds.

3. A compound of the formula (I) as claimed in claim 1, in which at least one of the radicals R₁ to R₄ denotes the group -CH₂-CH=CF-Rf (Rf = C₅F₁₁-C₁₁F₂₃), the group -CH₂-CH=CF-CₙF₂ₙ₊₁, where n = 5, 7 or 9, or the group -CH₂-CH=CF-C₇F₁₅, and not more than three of the radicals R₁ to R₄ independently of one another denote a methyl, ethyl, butyl or hydroxyethyl group, or a compound of the formula (II) mentioned in claim 1, in which Q, together with the constituent 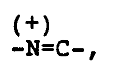 forms a pyridine, pyrazine or quinoline ring system, R₉ denotes a C₈F₁₇-CH₂-CH₂- group, R₁₀ denotes a hydrogen atom and the radicals R₅ to R₈ and R₁₁ to R₁₄ denote phenyl, p-chlorophenyl, p-toluyl or naphthyl radicals or fluorine atoms, or a mixture of these compounds.

4. A compound or mixture of compounds of the formula

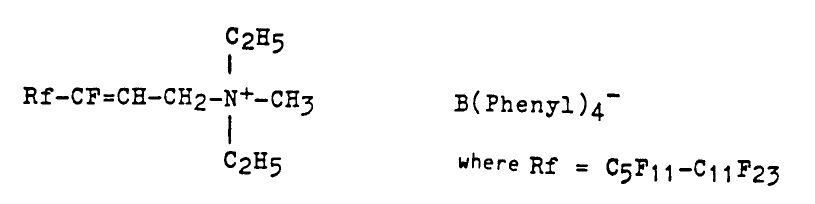

5. A compound or mixture of compounds of the formula

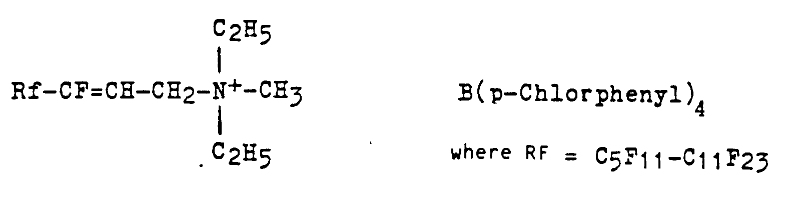

6. A compound or mixture of compounds of the formula

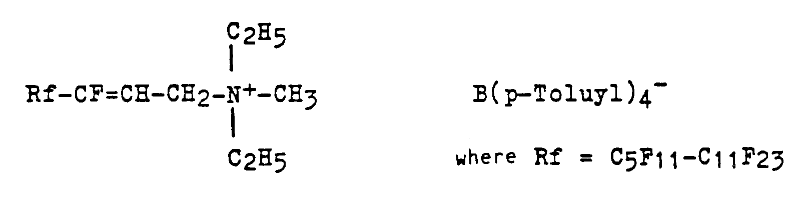

7. A compound or mixture of compounds of the formula

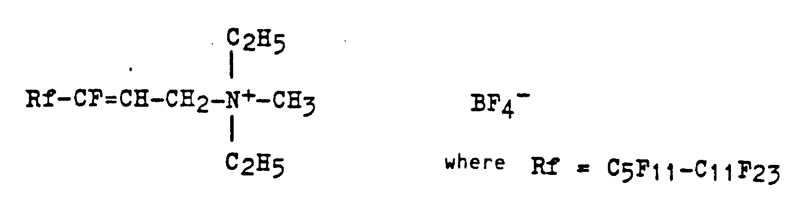

8. A compound or mixture of compounds of the formula

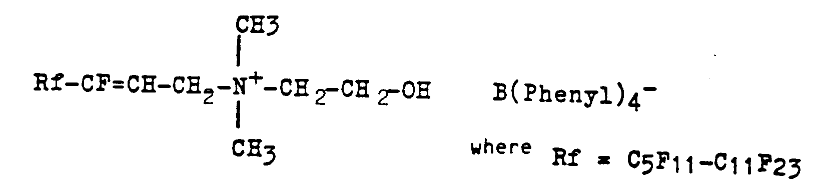

9. A compound or mixture of compounds of the formula

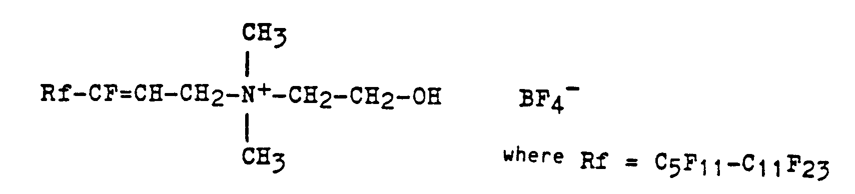

10. A compound or mixture of compounds of the formula

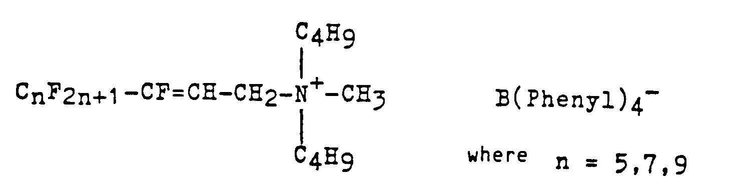

11. A compound or mixture of compounds of the formula

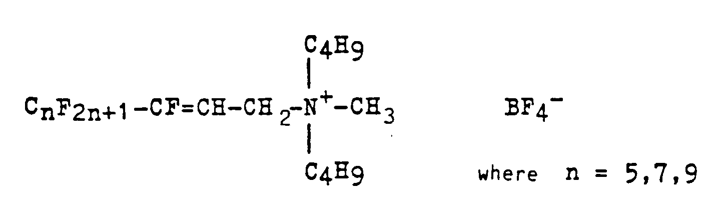

12. The compound of the formula

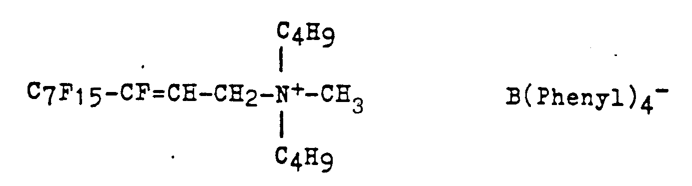

13. The compound of the formula

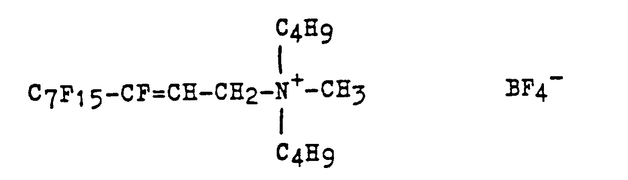

14. The compound of the formula

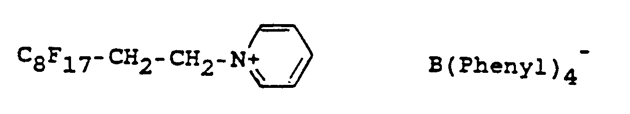

15. The compound of the formula

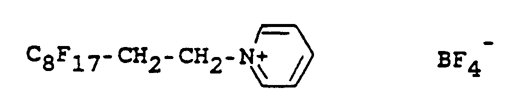

16. A process for the preparation of an ammonium compound or a mixture of ammonium compounds of the formula (I) as claimed in claim 1 or of an iminium compound or a mixture of iminium compounds of the formula (II) as claimed in claim 1, which comprises reacting an ammonium compound or a mixture of ammonium compounds of the formula (III) 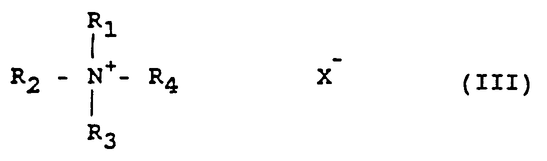 in which at least one of the radicals R₁ to R₄ denotes a straight-chain fluorine-containing unsaturated and/or saturated alkyl radical having 4 to 30 carbon atoms and not more than three of the radicals R₁ to R₄ independently of one another denote hydrogen atoms or straight-chain or branched alkyl or hydroxyalkyl radicals having 1 to 30 carbon atoms, and X⁻ denotes a halogen anion or a methylsulfate anion, or an iminium compound or a mixture of iminium compounds of the formula (IV) as claimed in claim 1 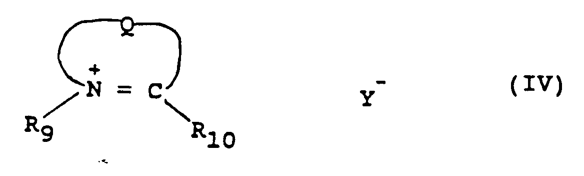 in which Q, together with the constituent 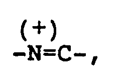 forms a mono- or polynuclear ring system having 4 to 17 carbon atoms, which can additionally be interrupted by 1 to 4 hetero atoms and contain 2 to 9 double bonds, and which can be substituted in any desired position by fluorine, chlorine, bromine or iodine atoms or an alkyl(C₁-C₆), alkoxy(C₁-C₆), nitro or amino group, R₉ denotes a fluorine-containing alkyl(C₁-C₃₀) radical, R₁₀ denotes a hydrogen, fluorine, chlorine, bromine or iodine atom or an alkyl(C₁-C₆), alkoxy(C₁-C₆), nitro or amino group and Y⁻ denotes a halogen anion or a methylsulfate anion, with a borate salt in water or mixtures of water and organic solvent at temperatures of about 20°C to about 90°C.

17. The process as claimed in claim 16, wherein X⁻ in the formula (III) mentioned therein and Y⁻ in the formula (IV) mentioned therein denote a chlorine, bromine or iodine anion.

18. The process as claimed in either of claims 16 and 17, wherein the reaction is carried out in a mixture of water and isopropanol, isobutanol or methyl isobutyl ketone.

19. The process as claimed in any one of claims 16 to 18, wherein the reaction is carried out with sodium tetraphenylborate, sodium tetra-p-chlorophenylborate, sodium tetra-p-toluylborate or sodium tetrafluoroborate as the borate salt.

## Revendications

1. Composés d'ammoniums répondant à la Formule générale (I) : 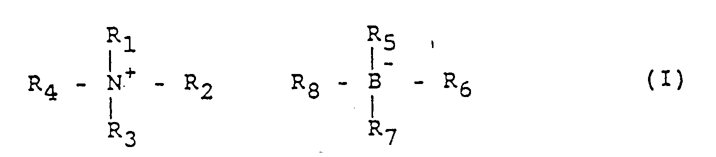 dans laquelle au moins un des symboles R₁ à R₄ représente un radical alkyle linéaire fluoré, saturé et/ou insature, qui contient de 4 à 30 atomes de carbone, et au plus trois des symboles R₁ à R₄ représentent chacun, indépendamment l'un de l'autre, un atome d'hydrogène ou un radical alkyle ou hydroxyalkyle linéaire ou ramifié contenant de 1 à 30, atomes de carbone, et, R₅ à R₈ représentent chacun un radical aryle, un radical alkylaryle, un radical halogéno-aryle ou un atome de fluor,
et composés d'immoniums répondant à la Formule générale (II) : 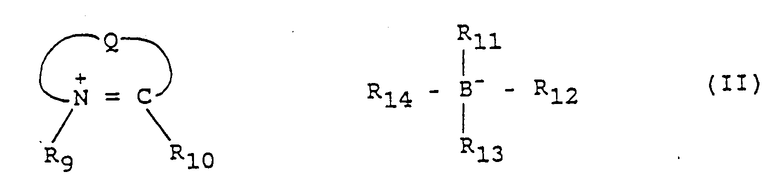 dans laquelle Q forme, avec la partie 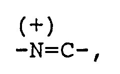 un système cyclique contenant de 4 à 17 atomes de carbone et comportant un ou plusieurs noyaux, système cyclique qui peut en outre être interrompu par 1 à 4 hétéro-atomes, qui peut contenir de 2 à 9 doubles liaisons et qui peut porter, en des positions quelconques, des atomes de fluor, de chlore, de brome ou d'iode ou des radicaux alkyles en C₁-C₆, alcoxy en C₁-C₆, nitro ou amino, R₉ représente un radical alkyle fluoré en C₁-C₃₀, R₁₀ représente un atome d'hydrogène, de fluor, de chlore, de brome ou d'iode ou un radical alkyle en C₁-C₆, alcoxy en C₁-C₆, nitro ou amino, et les symboles R₁₁ à R₁₄ représentent chacun un radical aryle, un radical alkylaryle, un radical halogénoaryle ou un atome de fluor,
ainsi que mélanges de ces composés.

2. Composés de Formule générale (I) selon la revendication 1, dans lesquels au moins un des symboles R₁ à R₄ représente un radical alkyle linéaire fluoré, insaturé et/ou saturé, contenant de 4 à 14 atomes de carbone, au plus trois des symboles R₁ à R₄ représentent chacun, indépendamment l'un de l'autre, un radical alkyle en C₁-C₄ ou hydroxyalkyle en C₁-C₄ linéaire ou ramifié, et R₅ à R₈ représentent chacun un radical phényle, naphtyle, p-chlorophényle ou p-toluyle ou un atome de fluor, ainsi que ceux des composés qui répondent à la Formule générale (II) mentionnée à la revendication 1 dans laquelle Q forme, avec la partie 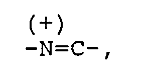 un système cyclique contenant de 4 à 10 atomes de carbone, à 1 ou à plusieurs noyaux, qui peut en outre être interrompu par 1 à 4 hétéro-atomes, qui peut contenir de 2 à 5 doubles liaisons et qui peut porter, en des positions quelconques, des atomes de fluor, de chlore, de brome ou d'iode ou des radicaux alkyles en C₁-C₆, alcoxy en C₁-C₆, nitro ou amino, R₉ représente un radical alkyle en C₁-C₄ qui contient du fluor, R₁₀ représente un atome d'hydrogène, de fluor, de chlore, de brome ou d'iode ou un radical alkyle en C₁-C₆, alcoxy en C₁-C₆, nitro ou amino, et les radicaux R₅ à R₈, et R₁₁ à R₁₄, représentent chacun un radical phényle, p-chlorophényle, p-toluyle ou naphtyle ou un atome de fluor, ainsi que mélanges de ces composés.

3. Composés de Formule générale (I) selon la revendication 1, dans lesquels au moins un des symboles R₁ à R₄ représente un radical -CH₂-CH=CF-Rf (Rf désignant -C₅F₁₁ à C₁₁F₂₃), un radical -CH₂-CH=CF-CₙF₂ₙ₊₁ dans lequel n désigne un nombre égal à 5, à 7 ou à 9, ou un radical -CH₂-CH=CF-C₇F₁₅, et au plus trois des symboles R₁ à R₄ représentent chacun, indépendamment l'un de l'autre, un radical méthyle, éthyle, butyle ou hydroxy-éthyle, ainsi que composés répondant à la Formule générale (II) mentionnée à la revendication 1 dans lesquels Q forme, avec forme, avec la partie 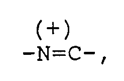 un système cyclique de la pyridine, de la pyrazine ou de la quinoléine, R₉ représente un radical C₈F₁₇-CH₂-CH₂-, R₁₀ représente un atome d'hydrogène et les symboles R₅ à R₈, et R₁₁ à R₁₄, représentent chacun un radical phényle, p-chlorophényle, p-toluyle ou naphtyle ou un atome de fluor, et mélanges de ces composés.

4. Composés ou mélanges de composés répondant à la Formule :

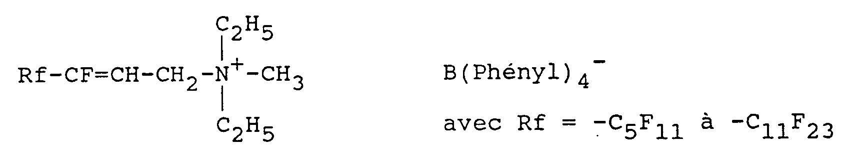

5. Composés ou mélanges de composés répondant à la Formule :

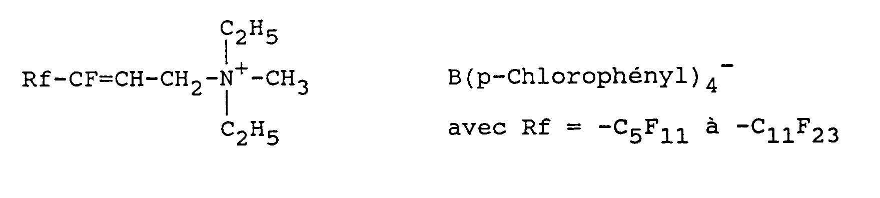

6. Composés ou mélanges de composés répondant à la Formule :

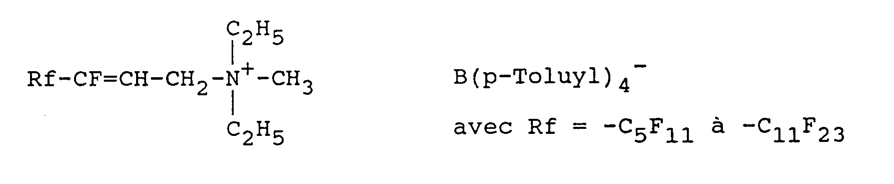

7. Composés ou mélanges de composés répondant à la Formule :

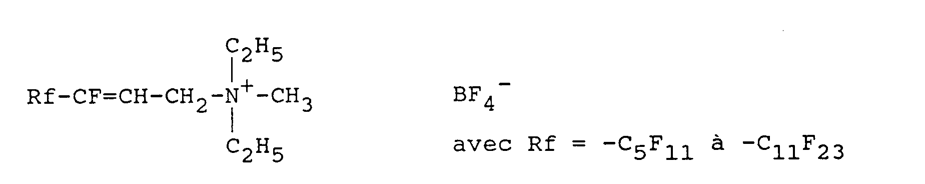

8. Composés ou mélanges de composés répondant à la Formule :

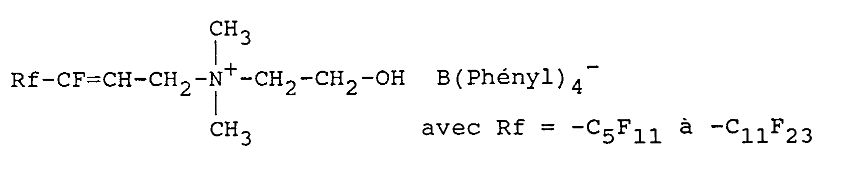

9. Composés ou mélanges de composés répondant à la Formule :

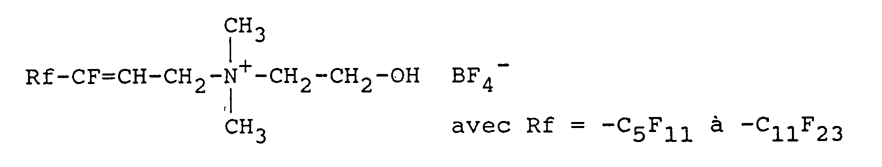

10. Composés ou mélanges de composés répondant à la Formule :

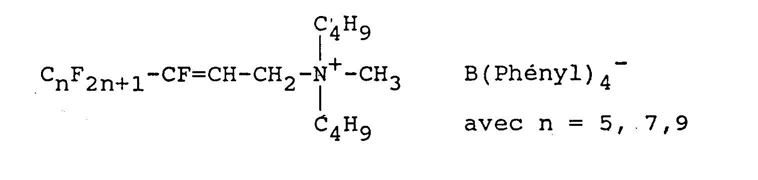

11. Composés ou mélanges de composés répondant à la Formule :

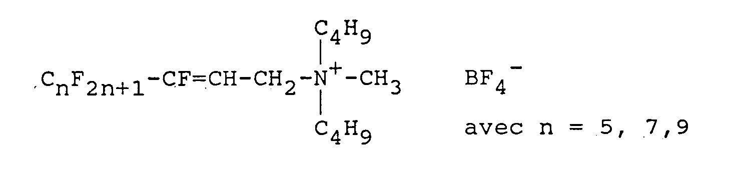

12. Composé de Formule :

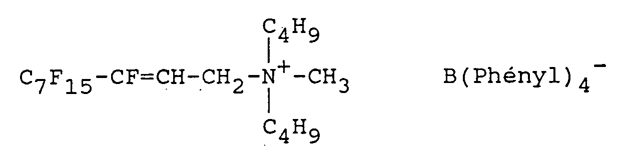

13. Composé de Formule :

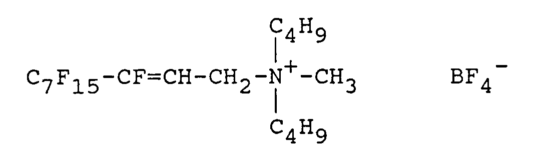

14. Composé de Formule :

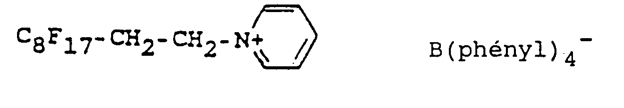

15. Composé de Formule :

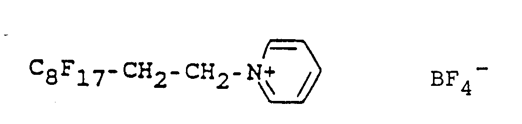

16. Procédé pour préparer des composés d'ammoniums ou des mélanges de composés d'ammoniums répondant à la Formule générale (I) définie à la revendication 1, ainsi que des composés d'immoniums ou des mélanges de composés d'immoniums répondant à la Formule générale (II) définie à la revendication 1, procédé caractérisé en ce qu'on fait réagir des composés d'ammoniums ou des mélanges de composés d'ammoniums répondant à la Formule générale (III) : 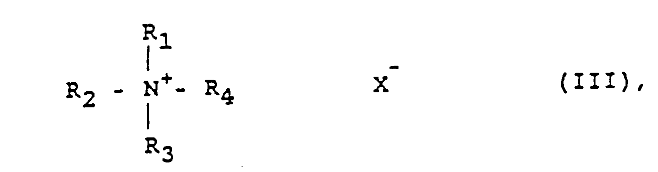 dans laquelle au moins un des symboles R1 à R4 représente un radical alkyle linéaire fluoré, insaturé et/ou saturé, contenant de 4 à 30 atomes de carbone, et au plus trois des symboles R₁ à R₄ représentent chacun, indépendamment l'un de l'autre, un atome d'hydrogène ou un radical alkyle ou hydroxyalkyle, linéaire ou ramifié, contenant de 1 à 30 atomes de carbone, et X⁻ représente un anion halogène ou un anion méthylsulfate,
ou des composés d'immoniums ou des mélanges de composés d'immoniums répondant à la Formule générale (IV) : 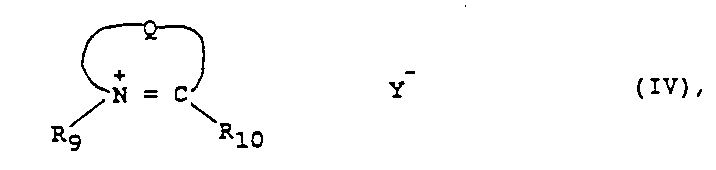 dans laquelle Q forme, avec la partie 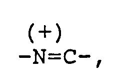 un système cyclique contenant de 4 à 17 atomes de carbone, à un ou à plusieurs noyaux, qui peut en outre être interrompu par 1 à 4 hétéro-atomes, qui peut contenir de 2 à 9 doubles liaisons et qui peut porter, en des positions quelconques, des atomes de fluor, de chlore, de brome ou d'iode ou des radicaux alkyles en C₁-C₆, alcoxy en C₁-C₆, nitro ou amino, R₉ représente un alkyle en C₁-C₃₀ contenant du fluor, R₁₀ représente un atome d'hydrogène, de fluor, de chlore, de brome ou d'iode ou un radical alkyle en C₁-C₆, alcoxy en C₁-C₆, nitro ou amino, et Y⁻ représente un anion halogène ou un anion méthylsulfate,
avec un sel d'acide borique, dans de l'eau ou dans des mélanges d'eau et d'un solvant organique, à des températures d'environ 20 à environ 90°C.

17. Procédé selon la revendication 16, caractérisé en ce que X⁻, dans la Formule générale (III) qui y est mentionnée, et Y⁻, dans la Formule générale (IV) qui y est mentionnée, représentent un atome de chlore, de brome ou d'iode.

18. Procédé selon l'une des revendications 16 et 17 caractérisé en ce qu'on effectue la réaction dans un mélange d'eau et d'isopropanol, d'isobutanol ou de méthyl-isobutyl-cétone.

19. Procédé selon l'une quelconque des revendications 16 à 18, caractérisé en ce qu'on effectue la réaction en utilisant, comme sel d'acide borique, le tétraphényl-borate de sodium, le tétrakis-(p-chlorophényl)-borate de sodium, le tétrakis-(p-toluyl)-borate de sodium ou le tétra-fluoroborate de sodium.
